# FASCICULE DE BREVET EUROPEEN

(11) **EP 0 815 810 B1**
(45) Date de publication et mention de la délivrance du brevet: **21.11.2001**
(21) Numéro de dépôt: 97420098.2
(22) Date de dépôt: 24.06.1997
(51) Int. Cl.: A61F 2/40

(54) **Jeu de tiges humérales pour une prothèse d'épaule**
Satz von Humerusschäften für eine Schulterprothese
Set of humeral shafts for a shoulder prosthesis

(30) Priorité: 25.06.1996 FR 9608152
(43) Date de publication de la demande: 07.01.1998
(73) Titulaire: TORNIER SA, F-38330 Saint Ismier (FR)
(72) Inventeur: Walch, Gilles, 69003 Lyon (FR); Boileau, Pascal, 06300 Nice (FR)
(74) Mandataire: Myon, Gérard Jean-Pierre

(56) Documents cités:
- EP-A- 0 477 113
- EP-A- 0 549 480
- EP-A- 0 679 375
- WO-A-94/15551
- DE-A- 2 041 929
- FR-A- 2 103 145
- FR-A- 2 380 021
- FR-A- 2 701 835

## Description

L'invention concerne un jeu de tiges humérales pour une prothèse de l'extrémité supérieure de l'humérus, comprenant une tige qui s'ancre dans le canal huméral et pourvue dans sa partie supérieure d'une face d'appui recevant une calotte hémisphérique propre à coopérer avec la glène de l'épaule.

On connaît des prothèses de ce genre qui comprennent une tige humérale comportant dans sa partie supérieure une face d'appui pourvue de moyen de blocage pour la fixation d'une calotte hémisphérique. On constate que la tige humérale présente une face d'appui unique propre à recevoir la calotte hémisphérique. Dans ces conditions, la calotte présente une inclinaison fixe et elle ne couvre pas de manière satisfaisante le plan de coupe de l'extrémité de l'humérus, de telle sorte qu'il faut adapter l'os à la prothèse avec toutes les conséquences anatomiques que cela entraîne.

De plus, on connaît, d'après la demande de brevet EP-A1-O 549 480 appartenant au Demandeur, une prothèse de l'extrémité supérieure de l'humérus permettant d'obtenir :
- un positionnement et un ancrage efficace dans le canal huméral ;
- la récupération de la hauteur totale de l'humérus reconstitué ;
- un diamètre de la calotte humérale propre à assurer une couverture satisfaisante du plan de coupe de l'humérus ;
- un angle d'inclinaison satisfaisant de la calotte humérale ;
- un angle de rétro-torsion de la calotte humérale convenable ;
- un déport latéral de la calotte par rapport à l'axe huméral ;
- un positionnement antéro-postérieur de la calotte par rapport à l'axe huméral ;
- et une hauteur appropriée de la calotte humérale.

Ainsi, la prothèse décrite dans la demande de brevet EP-A1-0 549 480 comprend une tige qui s'ancre dans le canal huméral, une calotte hémisphérique unique propre à coopérer avec la glène de l'épaule et une entretoise en forme de coin propre à faire varier la distance de la base de la calotte par rapport à la face d'appui de la tige. L'entretoise permet également de modifier l'orientation de la base de la calotte par rapport au plan contenant la face d'appui de la tige en changeant l'angle déterminé par ses faces extrêmes. En outre, cette prothèse comprend des moyens pour faire varier la position angulaire de la calotte autour d'un axe géométrique excentré par rapport à son propre axe géométrique.

Ce genre de prothèse comporte certains inconvénients en ce qui concerne l'agencement de plusieurs éléments indépendants, que le chirurgien doit assembler lors de l'opération afin d'obtenir une parfaite réparation de l'articulation de l'épaule. Cet agencement particulier est complexe. Il nécessite, de la part du chirurgien, des manipulations longues et difficiles pour obtenir le positionnement exact de la calotte hémisphérique par rapport à la coupe osseuse.

Le jeu de prothèses de l'extrémité supérieure de l'humérus suivant l'invention a pour but de donner au chirurgien la possibilité de couper le col huméral selon une ligne anatomique séparant clairement la partie articulaire de la métaphyse osseuse.

Dans cet esprit, l'invention concerne un jeu de prothèses de l'extrémité supérieure de l'humérus qui comprend plusieurs tiges pourvues chacune d'une face d'appui présentant un angle d'inclinaison différent par rapport à l'axe longitudinal de chaque tige.

Grâce à l'invention, le chirurgien n'est pas limité dans l'orientation qu'il donne à la coupe séparant la partie articulaire de la métaphyse osseuse. Il peut donc suivre précisément l'anatomie de l'épaule sur laquelle il intervient. Ensuite, le chirurgien choisit, en fonction de la taille du patient, un jeu de plusieurs tiges, par exemple 4 ou 6. Dans ce jeu, le chirurgien sélectionne alors la tige de prothèse qui s'adapte le mieux, en fonction de l'angle d'inclinaison de sa face d'appui, à la coupe préalablement réalisée afin de positionner exactement la calotte hémisphérique dans le plan frontal. Le jeu de prothèses suivant l'invention présente l'avantage supplémentaire que chaque tige peut être réalisée en une seule partie, ce qui est à comparer aux prothèses ajustables qui nécessitent plusieurs pièces à assembler en cours d'opération.

Selon un premier aspect avantageux de l'invention, la face d'appui de chaque tige est pourvue d'une collerette circulaire destinée à recevoir une calotte hémisphérique.

On peut également prévoir que la collerette circulaire de chaque tige s'engage à jeu réduit dans un emboîtement excentré de la base de la calotte hémisphérique. Cette structure permet un montage direct et aisé de la tige humérale sur la calotte sphérique.

Un jeu de tiges conforme à l'invention peut être prévu en plusieurs tailles correspondant aux différentes tailles possibles du patient, le diamètre de la diaphyse pouvant être compris entre 6 et 15 mm.

Selon un aspect avantageux du jeu de tiges de l'invention, une calotte hémisphérique unique est prévue pour coopérer avec la face d'appui de chaque tige. Ceci correspond au caractère modulaire de l'invention.

Selon un autre aspect avantageux de l'invention, la calotte hémisphérique unique comporte une base pourvue d'un emboîtement excentré dans lequel s'engage, à jeu réduit, une collerette circulaire constituant la face d'appui de chaque tige.

Dans ce cas, on peut prévoir que la collerette circulaire de chaque tige constituant le jeu de tiges est inclinée d'un angle différent par rapport à l'axe longitudinal de chaque tige.

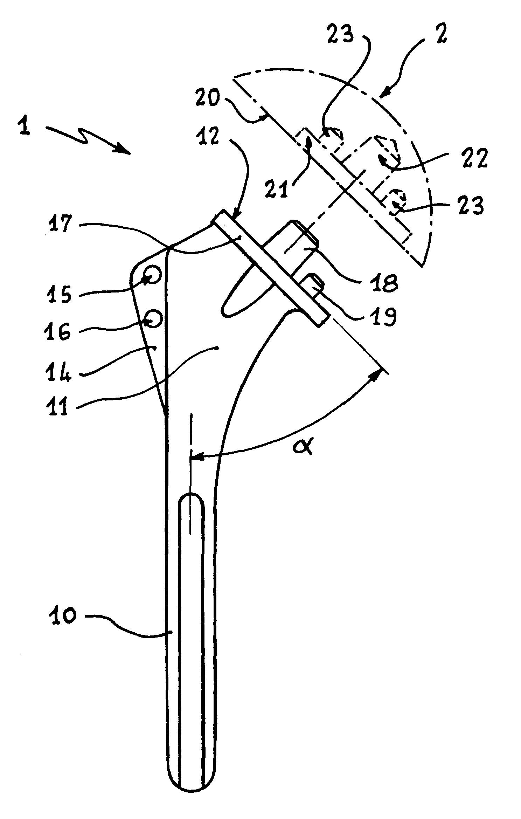

Le dessin annexé, donné à titre d'exemple, permettra de mieux comprendre l'invention, les caractéristiques qu'elle présente et les avantages qu'elle est susceptible de procurer.

La figure unique est une vue de face illustrant la prothèse de l'extrémité supérieure de l'humérus dont la face d'appui est inclinée d'un angle qui est différent pour chaque tige dans une taille donnée afin de s'adapter à la coupe osseuse réalisée par le chirurgien.

La figure unique montre une prothèse 1 de l'extrémité supérieure de l'humérus comportant une tige 10 qui s'ancre dans le canal huméral et qui est pourvue dans sa partie supérieure 11 d'une face d'appui 12 recevant une calotte hémisphérique 2 propre à coopérer avec la glène de l'épaule.

La tige 10 présente une section cylindrique qui s'engage dans le canal huméral et dont la partie supérieure ou métaphysaire 11 est conformée suivant un profil externe rectiligne. Dans le plan sagittal la partie métaphysaire 11 présente une légère conicité latérale de ses flancs. La partie métaphysaire ou supérieure 11 est solidaire d'un aileron 14 disposé sur la face externe de la tige 10 et qui comprend deux perforations 15, 16 permettant, dans le cas de fractures, une reconstitution de l'extrémité supérieure de l'humérus autour de la prothèse.

La tige 10 se termine à l'opposé de l'aileron 14 par une face d'appui 12 constituée par une collerette circulaire 17 qui est inclinée par rapport à l'axe longitudinal de la tige 10 d'un angle α. Cet angle α est fixe pour chaque tige 10 et différent entre les tiges d'un même jeu, de manière à constituer un jeu de tiges dont les faces d'appui 12 et leurs collerettes circulaires 17 respectives sont disposées dans des plans inclinés différents.

Ainsi, l'angle α peut être égal à 50° ou 45° ou 40° ou 35° pour chaque tige constituant le jeu de tiges humérales. Dans ce cas, le jeu de tiges comprend quatre tiges. Ce jeu peut être constitué de plusieurs tiges 10 ayant un angle α différent de ceux mentionnés ci-dessus, c'est-à-dire de plus de quatre tiges, pour augmenter le choix du chirurgien lorsque ce dernier a réalisé la coupe du col huméral suivant l'inclinaison nécessaire pour placer la calotte hémisphérique 2 dans une parfaite position dans le plan frontal. En effet, le chirurgien coupe le col huméral selon une ligne anatomique séparant clairement la partie articulaire de la métaphyse osseuse. Une fois la coupe réalisée, le chirurgien choisit la tige 10 dans le jeu de tiges pour que cette dernière s'adapte parfaitement à la coupe réalisée préalablement pour positionner exactement la calotte 2 dans le plan frontal.

En pratique, le jeu de tiges comprend, pour chaque taille de patient, entre 4 et 10 tiges.

La collerette circulaire 17 est solidaire en son milieu d'un pion tronconique 18 qui s'étend perpendiculairement à la face d'appui 12. A proximité du pion tronconique 18 est prévu un téton 19 qui s'étend perpendiculairement à la face d'appui 12.

La calotte hémisphérique 2 comporte une base 20 dans laquelle est ménagée de manière excentrée un emboîtement circulaire 21 dont le diamètre correspond au jeu près à celui de la collerette 17 de la face d'appui 12 de la tige d'appui 10. Au centre de l'emboîtement circulaire 21 est ménagé un alésage tronconique 22 de diamètre correspondant à celui du pion 18 de la partie métaphysaire 11 de la tige 10. Une série de trous 23 est réalisée dans le fond de l'emboîtement 21 de manière concentrique au centre de celui-ci. Le montage de la calotte hémisphérique 2 par rapport à la partie métaphysaire 11 de la tige 10 s'effectue par l'engagement du pion tronconique 18 dans l'alésage 22, la collerette circulaire 17 venant s'ajuster dans l'emboîtement 21, tandis que le téton 19 solidaire de la collerette 17 vient s'introduire dans l'un des trous 23 de la calotte hémisphérique 2. Bien entendu, par suite de la conicité d'environ 6° du pion 18 et de l'alésage 22, les deux éléments s'assemblent par coincement.

Comme indiqué précédemment, on dispose d'un jeu de tiges 10 comportant chacune une face d'appui 12 inclinée d'un angle α différent, de manière qu'on puisse régler l'inclinaison de la calotte hémisphérique 2 suivant la coupe osseuse préalablement réalisée par le chirurgien.

De même, on peut disposer d'un nombre conséquent de calottes hémisphériques 2 de diamètres différents à chacun desquels correspond une hauteur donnée en fonction du cas opératoire.

Grâce à la structure de la base de la calotte hémisphérique 2, on peut changer le positionnement antéro-postérieur de la calotte par rapport à l'axe huméral afin de s'adapter à toutes les positions nécessaire pour remplir la fonction normale de l'épaule traitée. De plus, grâce aux positions différentes de la calotte hémisphérique 2, on peut changer son positionnement anatomique de manière qu'elle puisse aussi bien être appliquée sur le côté gauche que sur le côté droit.

## Revendications

1. Jeu de tiges humérales pour prothèse de l'extrémité supérieure de l'humérus, **caractérisé en ce qu'**il comprend plusieurs tiges (10) pourvues chacune d'une face d'appui (12) présentant un angle (α) d'inclinaison différent par rapport à l'axe longitudinal de chaque tige (10).

2. Jeu de tiges humérales suivant la revendication 1, **caractérisé en ce qu'**une calotte hémisphérique unique (2) est prévue pour coopérer avec la face d'appui (12) de chaque tige (10).

3. Jeu de tiges humérales suivant la revendication 2, **caractérisé en ce que** la calotte hémisphérique unique (2) comporte une base (20) pourvu d'un emboîtement excentré (21) dans lequel s'engage à jeu réduit une collerette circulaire (17) constituant la face d'appui (12) de chaque tige (10).

4. Jeu de tiges humérales suivant la revendication 3, **caractérisé en ce que** la collerette circulaire (17) de chaque tige (10) constituant ledit jeu est inclinée d'un angle (α) différent par rapport à l'axe longitudinal de chaque tige (10).

5. Jeu de tiges humérales suivant la revendication 1, **caractérisé en ce que** ladite face d'appui (12) de chaque tige (10) est pourvue d'une collerette circulaire (17) propre à recevoir une calotte hémisphérique (2).

6. Jeu de tiges humérales suivant la revendication 5, **caractérisé en ce que** la collerette circulaire (17) de chaque tige (10) s'engage à jeu réduit dans un emboîtement excentré (21) de la base (20) de la calotte hémisphérique unique (2).

7. Jeu de tiges humérales suivant l'une des revendications 3 à 6, **caractérisé en ce que** la collerette circulaire (17) est solidaire en son milieu d'un pion tronconique (18) s'étendant perpendiculairement à la face d'appui (12) tandis qu'un téton (19) est disposé à proximité du pion tronconique (18).

8. Jeu de tiges humérales suivant l'une des revendications 2 à 7, **caractérisé en ce que** la calotte hémisphérique (2) comporte une base (20) dans laquelle est ménagé un emboîtement excentré (21) dont le fond est percé d'un trou borgne tronconique (22) et d'une série de trous concentriques (23) dans lesquels coopèrent respectivement le pion tronconique (18) et un téton (19) en vue de la fixation de la calotte (2) sur la partie métaphysaire (11) de la tige (10) correspondante.

9. Jeu de tiges humérales selon l'une des revendications précédentes, **caractérisé en ce que** chaque tige (10) est réalisée en une seule partie.

10. Jeu de tiges humérales selon l'une des revendications précédentes, **caractérisé en ce qu'**il est prévu en plusieurs tailles correspondant aux différentes tailles possibles du patient, le diamètre de la diaphyse pouvant être compris entre 6 et 15 mm.

## Patentansprüche

1. Oberarmstiftsatz für eine Prothese des oberen Endes des Oberarmknochens, **dadurch gekennzeichnet, dass** dieser mehrere Stifte (10) umfasst, wovon jeder mit einer Anlagefläche (12) versehen ist, die einen Neigungswinkel (α) aufweist, der sich gegenüber der Längsachse jedes Stifts (10) unterscheidet.

2. Oberarmstiftsatz nach Anspruch 1, **dadurch gekennzeichnet, dass** eine einzige halbkugelförmige Kappe (2) vorgesehen ist, um mit der Anlagefläche (12) jedes Stifts (10) zusammen zu wirken.

3. Oberarmstiftsatz nach Anspruch 2, **dadurch gekennzeichnet, dass** die einzige halbkugelförmige Kappe (2) einen Sockel (20) umfasst, der mit einer exzentrischen Ausnehmung (21) versehen ist, in die ein kreisförmiger Flansch (17) mit reduziertem Spiel eingreift, der die Anlagefläche (12) jedes Stifts (10) bildet.

4. Oberarmstiftsatz nach Anspruch 3, **dadurch gekennzeichnet, dass** der das Spiel bildende kreisförmige Flansch (17) jedes Stifts (10) um einen Winkel (α) geneigt ist, der sich gegenüber der Längsachse jedes Stifts (10) unterscheidet.

5. Oberarmstiftsatz nach Anspruch 1, **dadurch gekennzeichnet, dass** die Anlagefläche (12) jedes Stifts (10) mit einem kreisförmigen Flansch (17) versehen ist, der dazu geeignet ist, eine halbkugelförmige Kappe (2) aufzunehmen.

6. Oberarmstiftsatz nach Anspruch 5, **dadurch gekennzeichnet, dass** der kreisförmige Flansch (17) jedes Stifts (10) mit reduziertem Spiel in eine exzentrische Ausnehmung (21) des Sockels (20) der einzigen halbkreisförmigen Kappe (2) eingreift.

7. Oberarmstiftsatz nach einem der Ansprüche 3 bis 6, **dadurch gekennzeichnet, dass** der kreisförmige Flansch (17) in seiner Mitte fest mit einem kegelstumpfartigen Zapfen (18) verbunden ist, der sich senkrecht zu der Anlagefläche (12) erstreckt, während nahe bei dem kegelstumpfartigen Zapfen (18) ein Ansatz (19) vorgesehen ist.

8. Oberarmstiftsatz nach einem der Ansprüche 2 bis 7, **dadurch gekennzeichnet, dass** die halbkreisförmige Kappe (2) einen Sockel umfasst, in dem eine exzentrische Ausnehmung (21) ausgenommen ist, deren Boden von einem kegelstumpfartigen Grundloch (22) und einer Reihe von konzentrischen Löchern (23) durchbrochen ist, in denen jeweils der kegelstumpfförmige Zapfen (18) und der Ansatz (19) zur Befestigung der Kappe (2) am metaphysären Teil (11) des entsprechenden Stifts (10) zusammen wirken.

9. Oberarmstiftsatz nach einem der vorhergehenden Ansprüche, **dadurch gekennzeichnet, dass** jeder Stift (10) einstückig ausgebildet ist.

10. Oberarmstiftsatz nach einem der vorhergehenden Ansprüche, **dadurch gekennzeichnet, dass** er entsprechend den möglichen verschiedenen Grössen des Patienten in mehreren Grössen vorgesehen ist, wobei der Durchmesser der Diaphyse 6 bis 15 mm betragen kann.

## Claims

1. Set of humeral shafts for a prosthesis of the upper extremity of the humerus, **characterised in that** it comprises a plurality of rods (10), each provided with a supporting face (12) which presents a different angle of inclination (α) with respect to the longitudinal axis of each rod (10).

2. Set of humeral shafts according to claim 1, **characterised in that** a single hemispherical helmet (2) is provided to cooperate with the supporting face (12) of each rod (10).

3. Set of humeral shafts according to claim 2, **characterised in that** the single hemispherical helmet (2) has a base (20) provided with an off-centre nesting arrangement (21) in which a circular flanged portion (17) engages with little freeplay and constitutes the supporting face (12) of each rod (10).

4. Set of humeral shafts according to claim 3, **characterised in that** the circular flanged portion (17) of each rod (10) constituting said freeplay is inclined by a different angle (α) with respect to the longitudinal axis of each rod (10).

5. Set of humeral shafts according to claim 1, **characterised in that** said supporting face (12) of each rod (10) is provided with a circular flanged portion (17) adapted to receive a hemispherical helmet (2).

6. Set of humeral shafts according to claim 5, **characterised in that** the circular flanged portion (17) of each rod (10) engages with little freeplay in an off-centre nesting arrangement (21) in the base (20) of the single hemispherical helmet (2).

7. Set of humeral shafts according to any of claims 3 to 6, **characterised in that** the circular flanged portion (17) is rigidly joined at its centre to a tapered spigot (18) which extends perpendicularly to the supporting face (12) whereas a dog point (19) is disposed near to the tapered spigot (18).

8. Set of humeral shafts according to any of claims 2 to 7, **characterised in that** the hemispherical helmet (2) has a base (20) incorporating an off-centre nesting arrangement (21) the bottom of which is perforated by a truncated blind hole (22) and by a series of concentric holes (23) in which the tapered spigot (18) and a dog point (19) respectively cooperate in order to fix the helmet (2) on the metaphysial part (11) of the corresponding rod (10).

9. Set of humeral shafts according to any of the preceding claims, **characterised in that** each rod (10) is realised as a single part.

10. Set of humeral shafts according to any of the preceding claims, **characterised in that** it is provided in a plurality of sizes which correspond to the various possible sizes of patient, the diameter of the diaphysis possibly ranging from 6 to 15 mm.
